(19) Europäisches Patentamt
European Patent Office
Office européen des brevets

(11) EP 2 771 413 B1

(12) **FASCICULE DE BREVET EUROPEEN**

(45) Date de publication et mention
de la délivrance du brevet:
**22.11.2017 Bulletin 2017/47**

(21) Numéro de dépôt: **12781367.3**

(22) Date de dépôt: **08.10.2012**

(51) Int Cl.:
*C09C 1/28* $^{(2006.01)}$      *B01F 17/00* $^{(2006.01)}$
*D21H 17/00* $^{(2006.01)}$      *D21H 19/40* $^{(2006.01)}$
*D21H 19/58* $^{(2006.01)}$      *A61K 9/16* $^{(2006.01)}$

(86) Numéro de dépôt international:
**PCT/FR2012/052277**

(87) Numéro de publication internationale:
**WO 2013/060961 (02.05.2013 Gazette 2013/18)**

(54) **PROCÉDÉ DE FABRICATION DE SUSPENSIONS AQUEUSES DE TALC À PARTIR D'UN POLYMÈRE ACRYLIQUE À FONCTION TENSIO-ACTIVE GREFFÉE, SUSPENSIONS OBTENUES ET LEURS UTILISATIONS**

VERFAHREN ZUR HERSTELLUNG VON WÄSSRIGEN SUSPENSIONEN AUS TALK AUS EINEM ACRYLPOLYMER MIT EINER GEPFROPFTEN OBERFLÄCHENAKTIVEN FUNKTION, IN DIESEM VERFAHREN HERGESTELLTE SUSPENSIONEN UND VERWENDUNGEN DAVON

METHOD FOR MANUFACTURING AQUEOUS SUSPENSIONS OF TALC FROM AN ACRYLIC POLYMER HAVING A GRAFTED SURFACE-ACTIVE FUNCTION, RESULTING SUSPENSIONS, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.10.2011 FR 1159694**

(43) Date de publication de la demande:
**03.09.2014 Bulletin 2014/36**

(73) Titulaire: **COATEX**
**69730 Genay (FR)**

(72) Inventeurs:
• **JACQUEMET, Christian**
**F-69005 Lyon (FR)**
• **SUAU, Jean-Marc**
**F-69480 Lucenay (FR)**

(74) Mandataire: **Balmefrezol, Ludovic Francis Pierre et al**
**Coatex SAS**
**35, rue Ampère**
**69730 Genay (FR)**

(56) Documents cités:
**EP-A1- 0 892 020**      **FR-A1- 2 900 410**
**FR-A1- 2 913 426**      **US-B2- 6 946 510**

**Description**

La présente invention a trait à un nouveau procédé de mise en suspension du talc dans l'eau, à partir d'un additif polymérique permettant de fabriquer des suspensions aqueuses de talc présentant un compromis encore jamais égalé entre un extrait sec élevé, une stabilité améliorée, et une dose de dispersant réduite. Ce résultat est d'autant plus intéressant qu'on sait les nombreuses applications des suspensions aqueuses de talc, et la difficulté à mettre en suspension dans l'eau ce minéral particulièrement hydrophobe.

Le talc est un minéral utilisé dans de nombreuses applications. Dans l'industrie papetière il sert notamment comme charge, mais peut aussi être utilisé dans les sauces de couchage, ou en tant qu'additif permettant de réduire la quantité de matières collantes (pitch ou stickies) dans le procédé papetier ; il est aussi utilisé dans les peintures, dans les plastiques et les caoutchoucs chargés, dans les céramiques, et au sein des compositions alimentaires, cosmétiques et même dans l'agriculture.

Le talc peut être transporté sous forme de poudre, mais il est aussi mis en suspension dans l'eau pour des questions liées à la facilité de transport, de déchargement et de transvasement d'un produit liquide à la différence d'un produit pulvérulent. En outre, les poudres fines représentent aussi un danger pour l'homme, si elles sont inhalées en trop grande quantité. La Demanderesse précise que l'étape de mise en suspension du talc dont il est question, peut éventuellement être précédée d'une étape de granulation, cette dernière consistant à agglomérer des particules individuelles de talc de manière à former un granulé humide.

Ceci étant, le talc est une substance hautement hydrophobe, présentant une énergie de surface de l'ordre de 68 à 70 J/cm$^2$. Par conséquent, mettre en suspension dans l'eau une quantité importante de cette matière minérale, sans pour autant atteindre des niveaux de viscosité qui rendraient la suspension impropre au stockage ou à la manipulation représente un défi technique pour l'homme du métier. Ceci est notamment rapporté dans le document «The importance of surface energy in the dispersion behaviour of talc particles in aqueous media» (Powder Technology, 2009, 190 (1-2), pp. 242-246).

On est donc face à la double problématique d'augmenter ce qu'on appelle l'extrait sec (ou % en poids sec de talc présent dans la suspension aqueuse) de la suspension aqueuse de talc tout en conférant à celle-ci une rhéologie compatible avec son utilisation (c'est-à-dire en limitant l'augmentation de la viscosité de ladite suspension). Cette dernière viscosité peut être mesurée à partir de n'importe quelle technique familière à l'homme du métier, comme un rhéomètre Brookfield™, un dispositif de type coupe Ford™, etc...

Le choix de la mesure et du dispositif associé importe peu, dans la mesure où ils permettent d'appréhender le comportement rhéologique de la suspension au stockage et/ou à la manipulation. Cette indépendance est rappelée dans le document EP 1 074 293 A1 qui traite de manière très générale de la dispersion des matières minérales dans l'eau, le talc étant notamment cité parmi les substances à disperser.

Pour résoudre le problème évoqué plus haut, il est bien connu de mettre en oeuvre des agents dispersants, introduits pendant l'étape de mise en suspension du talc dans l'eau, et/ou pendant l'étape éventuelle de granulation qui la précède.

L'homme du métier connaît à ce sujet le document JP 63-175197 qui recommande la mise en oeuvre de polymères acryliques en mélange avec des esters de polylakylène glycol. On obtient une suspension d'un extrait sec voisin de 60 % ; son comportement rhéologique est étudié à travers un test de passage dans un tamis ; on observe un écoulement aisé et rapide de la suspension pour une maille du tamis égale à 150 $\mu$m. Cependant, la dose de dispersant sec utilisée se situe aux alentours de 10 % en poids par rapport au poids sec de talc : les performances doivent donc être relativisées à l'égard de cette quantité élevée de dispersant.

De même, le document JP 02-253836 préconise l'emploi d'un copolymère (méth)acrylique en combinaison avec un acide sulphosuccinique. Même si on arrive à un extrait sec de 65 % pour une viscosité Brookfield™ à 100 tours par minute comprise entre 300 et 680 mPa.s, la proportion de dispersant comprise entre 2 et 3 % en poids sec par rapport au poids sec de talc ne peut donner satisfaction à l'homme du métier.

On connaît aussi le document FR 2 380 065 A1 qui décrit des suspensions aqueuses de talc ayant des extraits secs supérieurs à 65 %, et des viscosités Brookfield™ à 100 tours par minute de l'ordre de 1000 mPa.s. Toutefois, les exemples démontrent que la quantité en poids sec de dispersant doit être supérieure à 1 % du poids sec de talc, préférentiellement de l'ordre de 3 %. Enfin, le dispersant utilisé est un sel de phénol, et plus particulièrement un alkyle phénol. Or, ces substances sont aujourd'hui prohibées dans l'industrie, notamment dans les peintures aqueuses, qui constituent une des applications potentielles des suspensions aqueuses de talc.

On connaît également les documents US 6 267 811 A1 et WO 2010 055191 A1 qui recommandent de mettre en oeuvre un système CMC (carboxyméthyl cellulose) / polyacrylate de sodium. Le niveau de performances atteint par ce couple reste toutefois modeste, pour ce qui est de la rhéologie de la suspension en fonction de son extrait sec. De plus, cette solution présente l'inconvénient de recourir à deux composants, ce qui multiplie par autant les installations de stockage et de transvasement (la cellulose modifiée agit ici comme un agent mouillant).

Une variante de la solution précédente réside dans la mise en oeuvre d'un polyacrylate de sodium avec un tensio-actif (GB 2019 822). Si on améliore les caractéristiques de la suspension aqueuse de talc résultante au sens de la présente invention, on observe alors la formation d'une mousse très abondante, facteur extrêmement nuisible à l'application finale, la mousse résultant de la présence du tensio-actif sous forme libre dans l'eau. On indique que les 2 solutions techniques polyacrylate/CMC et polyacrylate/tensio-actif ont fait l'objet de tests comparatifs dans la présente Demande.

Aujourd'hui, c'est encore la solution technique proposée dans le document EP 0 892 020 A1, pourtant vieux de plus de 10 ans, qui offre le meilleur compromis entre des performances viscosité/extrait sec et des taux de dispersants réduits et ce, sans produire de mousse. Cette solution repose sur la mise en oeuvre d'un copolymère hydrosoluble, constitué d'un premier monomère carboxylique (et notamment (méth)acrylique) et d'un second monomère porteur une fonction tensio-active greffée, ledit copolymère répondant à la formule suivante :

$$R - (OE)m - (OP)_n - R'$$

avec :

- m et n désignant des entiers inférieurs ou égaux à 100, dont un au moins est non nul,
- OE et OP désignant respectivement l'oxyde d'éthylène et l'oxyde de propylène,
- R désignant une fonction polymérisable, et préférentiellement la fonction méthacrylate ou méthacryluréthane,
- R' désignant un groupement linéaire ou ramifié alkyle, alkylaryle, arylalkyle, aryle ayant au moins 22 atomes de carbone ou une dialkylamine ayant au moins 22 atomes de carbone.

Les nombreux exemples soutenant cette invention démontrent qu'on peut fabriquer, avec seulement 1 % en poids sec par rapport au poids sec de talc des copolymères explicités ci-dessus, des suspensions aqueuses présentant un extrait sec en talc voisin de 65 % de leur poids, qui demeurent à la fois stables, manipulables et compatibles avec leurs utilisations finales. Les meilleurs résultats sont obtenus en choisissant un groupement aryle ayant 30 atomes de carbone.

Aujourd'hui, la Demanderesse qui a poursuivi ses efforts de recherche en vue d'améliorer le compromis extrait sec/viscosité d'une suspension aqueuse de talc et de limiter la quantité de dispersant mise en oeuvre, est parvenue à une solution qui va au-delà des performances décrites dans le document EP 0 892 020 A1. Cette solution est particulièrement originale puisqu'elle repose sur un copolymère de l'acide (méth)acrylique avec un monomère de formule similaire à celle décrite dans le document EP 0 892 020 A1, mais avec pour différence majeure le choix de R' : il s'agit dans la présente invention de l'hydrogène ou d'un groupe terminal alkyl linéaire ou ramifié ayant moins de 14 atomes de carbone.

Ce dernier monomère est donc beaucoup moins hydrophobe que son prédécesseur évoqué dans le document EP 0 892 020 A1 ; son caractère tensio-actif est lui aussi très largement diminué. Aussi, la solution mise au point par la Demanderesse est d'autant plus surprenante qu'elle s'oppose à un enseignement vieux de plus de dix ans et relaté dans le document précité qui recommandait un copolymère (méth)acrylique avec un monomère très fortement tensio-actif (disposant d'un groupe terminal ayant au moins 22 atomes de carbone) pour disperser efficacement du talc dans l'eau.

On montre alors clairement, à partir d'une mesure de viscosité de type coupe Ford™, que des suspensions aqueuses de talc présentant un extrait sec de plus de 60 % ont, dans le cadre de l'invention, une viscosité inférieure à celles fabriquées :

- selon le document EP 0 892 020 A1 (monomère tensio-actif ayant plus de 22 atomes de carbone) ;
- selon les documents US 6 267 811 A1 et WO 2010 055191 A1 (couple polyacrylate/CMC) ;
- et ne conduisent pas à la formation de mousse, contrairement à la solution recommandée dans le document GB 2 019 822 (couple polyacrylate/tensio-actif).

Ces résultats prennent tout leur sens à l'égard de l'art antérieur, dans la mesure où la quantité en poids sec de dispersant selon l'invention est inférieure à 1 % du poids total de talc sec.

Par ailleurs, le document US 6 946 510, concernant le secteur technique des charges minérales notamment pour les applications papetières, décrit l'utilisation d'un copolymère faiblement anionique et hydrosoluble, comme agent dispersant de pigments ou charges minérales en suspension aqueuse donnant, d'une part, un faible potentiel Zéta aux suspensions aqueuses desdites charges ou pigments et, d'autre part, apportant une stabilisation électro-stérique desdites suspensions.

Aussi, un premier objet de l'invention consiste en un procédé de fabrication d'une suspension aqueuse de talc, comprenant une étape de mise en suspension dans l'eau d'un talc, ledit talc résultant éventuellement d'une étape préalable de granulation, caractérisé en ce qu'on met en oeuvre, pendant l'étape de mise en suspension et/ou pendant l'étape éventuelle de granulation, au moins un polymère hydrosoluble, caractérisé en ce qu'il est constitué :

a) d'au moins un monomère (méth)acrylique, et
b) d'au moins un monomère de formule (I) :

$$R - (OE)m - (OP)_n - R' \qquad (I)$$

avec :

- m et n désignant des entiers inférieurs ou égaux à 115, dont un au moins est non nul,
- OE et OP désignant respectivement l'oxyde d'éthylène et l'oxyde de propylène, étant disposé de manière régulière ou aléatoire,
- R désignant une fonction polymérisable, choisie parmi la fonction acrylate, méthacrylate, méthacryluréthane, vinyl ou allyl,
- R' désignant l'hydrogène.

Un autre objet de la présente invention consiste en une suspension aqueuse de talc dans l'eau, caractérisée en ce qu'elle contient au moins un polymère hydrosoluble caractérisé en ce qu'il est constitué :

a) d'au moins un monomère (méth)acrylique, et
b) d'au moins un monomère de formule (I) :

$$R - (OE)m - (OP)_n - R' \qquad (I)$$

avec :

- m et n désignant des entiers inférieurs ou égaux à 115, dont un au moins est non nul,
- OE et OP désignant respectivement l'oxyde d'éthylène et l'oxyde de propylène, étant disposé de manière régulière ou aléatoire,
- R désignant une fonction polymérisable, choisie parmi la fonction acrylate, méthacrylate, méthacryluréthane, vinyl ou allyl,
- R' désignant l'hydrogène.

Selon un mode de réalisation de la présente invention, le procédé ou la suspension sont aussi caractérisés en ce que ledit polymère hydrosoluble est constitué de, exprimé en % en poids de chacun de ses monomères :

a) au moins 60 % d'au moins un monomère (méth)acrylique,
b) au plus 40 % d'au moins un monomère de formule (I).

Dans une première variante de réalisation de la présente invention, pour le monomère de formule (I), les entiers m et n sont non nuls et compris entre 10 et 75.
Selon la présente invention, la fonction R' du monomère de formule (I) représente l'hydrogène.
Selon un autre mode de réalisation, ledit polymère hydrosoluble est constitué de, exprimé en % en poids de chacun de ses monomères :

a) de 60 % à 99 % d'au moins un monomère (méth)acrylique, et
b) de 1 % à 40 % d'un monomère de formule (I).

Selon un mode de réalisation de la présente invention, ledit polymère hydrosoluble présente une viscosité spécifique comprise entre 1 et 50, telle que mesurée selon la méthode décrite dans le document EP 0 892 020 A1.
Le terme « viscosité spécifique » dans la signification de la présente invention est défini comme la différence de la viscosité relative mesurée à une température déterminée (par exemple 20°C ou 25°C) moins 1.

$$\eta_{sp} = \eta_{rel} - 1$$

La viscosité relative utilisée ici est le quotient de la viscosité de la solution $\eta$ et de la viscosité du solvant $\eta_0$

$$\eta_{rel} = \frac{\eta}{\eta_0}$$

où la viscosité du solvant $\eta_0$ est définie comme la viscosité du solvant pur à une température déterminée (par exemple 20°C ou 25°C) et la viscosité de la solution $\eta$ est définie comme la viscosité du copolymère en peigne dissous dans le solvant pur à une température déterminée (par exemple 20°C ou 25°C) et à une concentration en polymère déterminée (par exemple 45 g/l).

Cependant, pour déterminer la viscosité relative, il est suffisant de mesurer le temps d'élution t (de la solution) et $t_0$ (du solvant) à une température donnée (par exemple 20°C ou 25°C) si les conditions aux limites sont constantes. Par conséquent, la viscosité relative peut être définie par :

$$\eta_{rel} = \frac{t}{t_0}$$

et donc, la viscosité spécifique peut être définie par :

$$\eta_{sp} = \frac{t}{t_0} - 1.$$

La viscosité spécifique du polymère peut par exemple être déterminée par un système Lauda PVS, sur des solutions aqueuses de polymère (concentration en polymère déterminée par exemple de 45 g/l ou 50 g/l). Les mesures peuvent notamment être effectuées sur un tube capillaire Hubbelohde DIN (Schott) de type I (K = 0,010).

Selon un mode de réalisation de la présente invention, ledit polymère hydrosoluble est totalement ou partiellement neutralisé, préférentiellement par les ions sodium, calcium, potassium, magnésium, ammonium ou leurs mélanges.

Selon un autre mode de réalisation de la présente invention, ladite suspension de talc dans l'eau contient entre 0,001 % et 2 %, préférentiellement entre 0,1 % et 1 %, très préférentiellement entre 0,1 % et 0,75 %, en poids sec dudit polymère hydrosoluble par rapport au poids sec de talc.

Selon un autre mode encore de réalisation de la présente invention, ladite suspension de talc dans l'eau contient plus de 55 %, préférentiellement plus de 60 %, très préférentiellement entre 61 % et 65 % en poids sec de talc par rapport à son poids total.

Un autre objet de la présente invention réside dans l'utilisation de ladite suspension aqueuse pour fabriquer une sauce de couchage papetière, un additif permettant de réduire la quantité de matières collantes, une peinture, un plastique, un caoutchouc, une céramique, une composition alimentaire, une composition cosmétique, une formulation agricole.

Un dernier objet de la présente invention consiste en une sauce de couchage papetière, un additif permettant de réduire la quantité de matières collantes, une peinture, un plastique, un caoutchouc, une céramique, une composition alimentaire, une composition cosmétique, une formulation agricole, caractérisé en ce qu'il/elle contient un talc et un polymère tel que décrit ci-dessus.

## EXEMPLES

### Exemple 1

Cet exemple porte sur la mise en suspension d'un talc qui est le Mistron 85 commercialisé par la société Talc de Luzenac™.

On a cherché ici à réaliser des suspensions présentant un extrait sec en talc égal à 61 % de leur poids total.

A l'aide du moteur VMI Supertest™ (Rayneri™), on réalise une dispersion de talc à 61 % d'extrait sec.

Dans un bêcher en inox on commence par peser l'eau nécessaire afin d'avoir une concentration finale de 61 %. On ajoute ensuite le polymère selon l'invention ou le système polymère/CMC ou polymère/tensio-actif à tester (les quantités sont indiquées par la suite, pour chaque essai).

Le pH est ajusté à 12 avec une solution de soude à 50 %.

Le talc est ensuite versé dans le bêcher, petit à petit.

On augmente alors la vitesse d'agitation du moteur jusqu'à 2 500 tours / minute.

On laisse sous agitation à cette vitesse pendant 20 minutes.

On laisse refroidir la suspension jusqu'à 25°C.

Le pH est à nouveau ajusté à 11 avec une solution de soude à 50 %.

Pour chacune des suspensions obtenues, on détermine en seconde la valeur de sa viscosité coupe Ford™ selon la méthode bien connue de l'homme du métier (norme ISO 2431-1984 en utilisant une coupe Ford™ n° 4).

On évalue de manière visuelle la présence de mousse.

Essai n° 1

Cet essai illustre l'art antérieur, et plus précisément une solution du type polyacrylate/CMC, selon les documents US 6 267 811 A1 et WO 2010 055191 A1.

Il met en oeuvre par rapport au poids sec de talc, 0,35 % en poids sec d'un polyacrylate de sodium de masse moléculaire moyenne en poids égale à 3 800 g/mol et 0,35 % en poids sec d'une CMC (Finnfix-5™ commercialisé par la société CP-Kelko Oy™).

Essai n° 2

Cet essai illustre l'art antérieur et plus précisément une variante des documents US 6 267 811 A1 et WO 2010 055191 A1 à base d'un couple polyacrylate/tensio-actif.

Il met en oeuvre, par rapport au poids sec de talc, 0,1 % en poids sec d'un polyacrylate de sodium de masse moléculaire moyenne en poids égale à 3 800 g/mol et 0,9 % en poids sec d'un tensio-actif (Lumiten™ PR8709 commercialisé par la société BASF™).

Essai n° 3

Cet essai illustre l'art antérieur, et plus précisément une solution telle que décrite dans le document EP 0892020 A1, où le monomère tensio-actif est porteur d'un groupe hydrophobe terminal qui est le groupement alkyle linéaire ayant 22 atomes de carbone.

Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 79,5 % d'acide acrylique,
b) 20,5 % d'un monomère de formule (I) avec R désignant la fonction méthacrylate, n = 0 et m = 25, et R' est le groupe alkyle linéaire ayant 22 atomes de carbone.

Essai n° 4

Cet essai illustre l'art antérieur, et plus précisément une solution telle que décrite dans le document EP 0892020 A1, où le monomère tensio-actif est porteur d'un groupe hydrophobe terminal qui est un groupement aryle ayant 30 atomes de carbone, ledit groupement étant le tristyrylphénol.

Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 74,0 % d'acide acrylique,
b) 26,0 % d'un monomère de formule (I) avec R désignant la fonction méthacryluréthanne, n = 0 et m = 40, et R' est le groupe tristyrylphénol ayant 30 atomes de carbone.

Essai n° 5

Cet essai illustre l'art antérieur, et plus précisément une solution telle que décrite dans le document EP 0892020 A1, où le monomère tensio-actif est porteur d'un groupe hydrophobe terminal qui est un groupement aryle ayant 30 atomes de carbone, ledit groupement étant le tristyrylphénol.

Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 76,0 % d'acide acrylique,
b) 24,0 % d'un monomère de formule (I) avec R désignant la fonction méthacrylate, n = 0 et m = 25, et R' est le groupe tristyrylphénol ayant 30 atomes de carbone.

**EP 2 771 413 B1**

Essai n° 6

Cet essai illustre un domaine n'appartenant pas à l'invention ni à l'art antérieur ; il illustre plus précisément une solution telle que décrite dans le document EP 0 892 020 A1, mais avec un monomère tensio-actif porteur d'un groupe hydrophobe terminal qui est le groupement alkyle linéaire ayant 16 atomes de carbone.
Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 81,0 % d'acide acrylique,
b) 19,0 % d'un monomère de formule (I) avec R désignant la fonction méthacryluréthanne, n = 0 et m = 25, et R' est le groupe alkyle linéaire ayant 16 atomes de carbone.

Essai n° 7

Cet essai illustre l'invention.
Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 75,0 % d'acide acrylique,
b) 25,0 % d'un monomère de formule (I) avec R désignant la fonction méthacrylate, n = 40 et m = 16, et R' est l'hydrogène.

Essai n° 8

Cet essai illustre l'invention.
Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 70,0 % d'acide acrylique,
b) 30,0 % d'un monomère de formule (I) avec R désignant la fonction méthacrylate, n = 48 et m = 16, et R' est l'hydrogène.

Essai n° 9

Cet essai illustre l'invention.
Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 65,0 % d'acide acrylique,
b) 35,0 % d'un monomère de formule (I) avec R désignant la fonction méthacrylate, n = 48 et m = 16, et R' est l'hydrogène.

Essai n° 10

Cet essai illustre l'invention.
Il met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

a) 60,0 % d'acide acrylique,
b) 40,0 % d'un monomère de formule (I) avec R désignant la fonction méthacrylate, n = 48 et m = 16, et R' est l'hydrogène.

Essai n° 11

Cet essai met en oeuvre, par rapport au poids sec de talc, 0,5 % en poids sec d'un polymère (dont 10 % molaire des sites carboxyliques sont neutralisés par l'ion sodium) constitué de, exprimé en % en poids de chacun de ses constituants :

7

a) 77,0 % d'acide acrylique,
b) 23,0 % d'un monomère de formule (I) avec R désignant la fonction méthacrylate, n = 0 et m = 45, et R' est le groupe méthyle.

Essai n° 12

Cet essai met en oeuvre, par rapport au poids sec de talc, 0,5 % en poids sec d'un polymère (dont 50 % molaire des sites carboxyliques sont neutralisés par l'ion potassium) constitué de, exprimé en % en poids de chacun de ses constituants :

a) 77,0 % d'acide acrylique,
b) 23,0 % d'un monomère de formule (I) avec R désignant la fonction méthacrylate, n = 0 et m = 113, et R' est le groupe méthyle.

| Essai n° | 1 | 2 | 3 | 4 | 5 |
|---|---|---|---|---|---|
| Art Antérieur INvention Hors Invention | AA | AA | AA | AA | AA |
| Additif* | polyacrylate CMC | polyacrylate tensio-actif | polymère tensio-actif | polymère tensio-actif | polymère tensio-actif |
| R' | - | - | alkyle C22 | TSP C30 | TSP C30 |
| Viscosité coupe Ford™ (s) | 253 | 80** | 286 | 171 | 124 |

| Essai n° | 6 | 7 | 8 | 9 | 10 |
|---|---|---|---|---|---|
| Art Antérieur INvention Hors Invention | HI | IN | IN | IN | IN |
| additif | polymère tensio-actif | polymère tensio-actif | polymère tensio-actif | polymère tensio-actif | polymère tensio-actif |
| R' | alkyle C16 | H | H | H | H |
| Viscosité coupe Ford™ (s) | 131 | 98 | 92 | 75 | 97 |

| Essai n° | 11 | 12 |
|---|---|---|
| Art Antérieur INvention Hors Invention | HI | HI |
| additif | polymère tensio-actif | polymère tensio-actif |
| R' | alkyle C1 | alkyle C1 |
| Viscosité coupe Ford™ (s) | 90 | 110 |

**Tableau 1**

* additif désigne le système mis en œuvre pour disperser (et stabiliser la suspension) à base d'un couple polyacrylate/CMC ou polyacrylate/tensio-actif ou d'un polymère tensio-actif

** présence de mousse très abondante

La lecture du tableau 1 démontre que le système polyacrylate/tensio-actif selon l'essai n° 1 est réellement peu performant : il conduit à la valeur de viscosité coupe Ford™ élevée.

Inversement, le couple polyacrylate/tensio-actif selon l'essai n° 2 pourrait constituer une solution intéressante, puisqu'associé à une valeur de viscosité coupe Ford™ de 80 secondes seulement. Néanmoins, on observe de manière très claire la formation d'une mousse extrêmement abondante. Or, une telle mousse est absolument incompatible avec la mise en oeuvre de la suspension pour fabriquer un produit final, comme une peinture, une sauce de couchage papetière, etc... Il faut ajouter qu'aucun des 11 autres essais n'a conduit à la formation de mousse.

Les essais n° 3, 4 et 5 qui correspondent à un polymère dont le monomère tensio-actif dispose de plus de 22 atomes de carbone sur son groupement terminal, conduisent systématiquement à des valeurs plus élevées de viscosité coupe Ford™ (plus de 120 secondes) que pour les polymères de l'invention (moins de 110 secondes).

Enfin, l'essai n° 6 qui illustre la présence d'un groupe alkyle ayant 16 atomes de carbone dans le monomère tensio-actif conduit à une valeur de viscosité coupe Ford™ égale à 131, ce qui n'est pas non plus satisfaisant.

En résumé, seuls les polymères selon l'invention (essais n° 7 à 10) conduisent à la fois aux valeurs de viscosité coupe Ford™ les moins élevées (inférieures à 110 secondes) et ce, sans provoquer la formation de mousse, et avec une faible dose de dispersant.

**Exemple 2**

Cet exemple porte sur la mise en suspension d'un talc purifié par flottation qui est le Finntalc™ C10 commercialisé par la société Mondo Minerais™.

Cet exemple illustre la capacité du dispersant selon l'invention à mettre en suspension un autre type de talc que le précédent : il sert à illustrer le caractère « universel » dudit dispersant.

On a cherché ici à réaliser des suspensions présentant un extrait sec en talc égal à 65 % de leur poids total.

Dans un bêcher en inox, on pèse l'eau nécessaire afin d'avoir une concentration finale de 68 % en poids sec de talc dans l'eau.
On ajoute ensuite le polymère ou le système polymère/CMC ou polymère/tensio-actif à tester (les quantités sont indiquées par la suite, pour chaque essai).
Le pH de la solution est ajusté à 12 au moyen d'une solution de soude à 50 %.
On introduit la solution aqueuse dans un disperseur Lödige ; après avoir mis en route le malaxeur, on introduit petit à petit le talc dans le disperseur. La turbine assurant un fort cisaillement est ensuite démarrée et on laisse tourner pendant 1 heure en régulant la température de la suspension entre 30 et 40°C.
La suspension est alors diluée à un extrait sec de 65 % en talc. Son pH est ajusté à 10 au moyen d'une solution de soude à 50 %.

Pour chacune des suspensions obtenues, on détermine en seconde la valeur de sa viscosité coupe Ford™ selon la méthode bien connue de l'homme du métier (norme ISO 2431-1984 en utilisant une coupe Ford™ n° 4).
On évalue de manière visuelle la présence de mousse.

Essai n° 13

Cet essai illustre l'art antérieur, et plus précisément une solution du type polyacrylate/CMC, selon les documents US 6 267 811 A1 et WO 2010 055191 A1.

Il met en oeuvre par rapport au poids sec de talc, 0,35 % en poids sec d'un polyacrylate de sodium de masse moléculaire moyenne en poids égale à 3 800 g/mol et 0,35 % en poids sec d'une CMC (Finnfix-5™ commercialisé par la société CP-Kelko Oy™). Pour cet essai on est obligé de commencer à disperser à 65 % d'extrait sec, puis de diluer à un extrait sec final de 61 % en poids sec de talc sans quoi, il est impossible d'obtenir une suspension manipulable.

Essai n° 14

Cet essai illustre l'art antérieur et plus précisément une variante des documents US 6 267 811 A1 et WO 2010 055191 A1 à base d'un couple polyacrylate/tensio-actif. Il met en oeuvre, par rapport au poids sec de talc, 0,15 % en poids sec d'un polyacrylate de sodium de masse moléculaire moyenne en poids égale à 3 800 g/mol et 1,5 % en poids sec d'un tensio-actif (Lumiten™ PR8709 commercialisé par la société BASF™).

Essai n° 15

Cet essai illustre l'invention.

Il met en oeuvre, par rapport au poids sec de talc, 0,6 % en poids sec d'un polymère (dont 50 % molaire des sites carboxyliques sont neutralisés par l'ion sodium) constitué de, exprimé en % en poids de chacun de ses constituants :

a) 65,0 % d'acide acrylique,
b) 35,0 % d'un monomère de formule (I) avec R désignant la fonction méthacrylate, n = 48 et m = 16, et R' est l'hydrogène.

Essai n° 16

Cet essai met en oeuvre, par rapport au poids sec de talc, 0,6 % en poids sec d'un polymère (dont 50 % molaire des sites carboxyliques sont neutralisés par l'ion sodium) constitué de, exprimé en % en poids de chacun de ses constituants :

a) 77,0 % d'acide acrylique,
b) 23,0 % d'un monomère de formule (I) avec R désignant la fonction méthacrylate, n = 0 et m = 45, et R' est le groupe méthyle.

**Tableau 2**

| Essai n° | 13 | 14 | 15 | 16 |
|---|---|---|---|---|
| Art Antérieur INvention | AA | AA | IN | HI |
| Additif* | polyacrylate CMC | polyacrylate tensio-actif | polymère tensio-actif | polymère tensio-actif |
| R' | - | - | H | alkyle Cl |
| Viscosité coupe Ford™ (s) | 305 | 100** | 95 | 105 |
| * additif désigne le système mis en oeuvre pour disperser (et stabiliser la suspension) à base d'un couple polyacrylate/CMC ou polyacrylate/tensio-actif ou d'un polymère tensio-actif ** mousse très abondante | | | | |

La lecture du tableau 2 démontre que le système polyacrylate/CMC selon l'essai n° 13 est réellement peu performant : il conduit à la valeur de viscosité coupe Ford™ la plus élevée.
Inversement, le couple polyacrylate/tensio-actif selon l'essai n° 14 pourrait constituer une solution intéressante (il faut cependant noter que la dose en poids sec d'additifs mis en oeuvre est ici égale à 1,65 % du poids sec de talc, par rapport aux 0,6 % seulement de polymère de l'invention). De plus, on observe de manière très claire la formation d'une mousse extrêmement abondante. Aucun des autres essais n'a engendré de mousse.
L'essai n° 15 qui correspond à un polymère selon l'invention conduit à la fois aux valeurs de viscosité coupe Ford™ parmi les moins élevées et ce, sans provoquer la formation de mousse, et avec une faible dose de dispersant.

**Revendications**

1. Procédé de fabrication d'une suspension aqueuse de talc, comprenant une étape de mise en suspension dans l'eau d'un talc, ledit talc résultant éventuellement d'une étape préalable de granulation, **caractérisé en ce qu'**on met en oeuvre, pendant l'étape de mise en suspension et/ou pendant l'étape éventuelle de granulation, au moins un polymère hydrosoluble, **caractérisé en ce qu'**il est constitué :

a) d'au moins un monomère (méth)acrylique, et
b) d'au moins un monomère de formule (I) :

$$R - (OE)m - (OP)_n - R' \qquad (I)$$

avec :

- m et n désignant des entiers inférieurs ou égaux à 115, dont un au moins est non nul,
- OE et OP désignant respectivement l'oxyde d'éthylène et l'oxyde de propylène,
- R désignant une fonction polymérisable choisie parmi la fonction acrylate, méthacrylate, méthacryluréthane, vinyl ou allyl,
- R' désignant l'hydrogène.

**2.** Procédé selon la revendication 1, **caractérisé en ce que**, pour le monomère de formule (I) les entiers m et n sont non nuls et compris entre 10 et 75.

**3.** Suspension aqueuse de talc dans l'eau, **caractérisée en ce qu'**elle contient au moins un polymère hydrosoluble constitué :

a) d'au moins un monomère (méth)acrylique, et
b) d'au moins un monomère de formule (I) :

$$R - (OE)m - (OP)_n - R' \qquad (I)$$

avec :

- m et n désignant des entiers inférieurs ou égaux à 115, dont un au moins est non nul,
- OE et OP désignant respectivement l'oxyde d'éthylène et l'oxyde de propylène,
- R désignant une fonction polymérisable, choisie parmi la fonction acrylate, méthacrylate, méthacryluréthane, vinyl ou allyl,
- R' désignant l'hydrogène.

**4.** Suspension aqueuse de talc dans l'eau selon la revendication 3, **caractérisée en ce que** ledit polymère hydrosoluble est constitué de, exprimé en % en poids de chacun de ses monomères :

a) au moins 60 % d'au moins un monomère (méth)acrylique,
b) au plus 40 % d'au moins un monomère de formule (I).

**5.** Suspension aqueuse de talc dans l'eau selon l'une quelconque des revendications 3 et 4, **caractérisée en ce que** ledit polymère hydrosoluble est constitué de, exprimé en % en poids de chacun de ses monomères :

a) de 60 % à 99 % d'au moins un monomère (méth)acrylique, et
b) de 1 % à 40 % d'un monomère de formule (I).

**6.** Suspension aqueuse de talc dans l'eau selon l'une des revendications 3 à 5, **caractérisée en ce que**, pour le monomère de formule (I) dudit polymère hydrosoluble, m et n sont non nuls et compris entre 10 et 75.

**7.** Suspension aqueuse de talc dans l'eau selon l'une des revendications 3 à 6, **caractérisée en ce que** ledit polymère hydrosoluble présente une viscosité spécifique comprise entre 1 et 50.

**8.** Suspension aqueuse de talc dans l'eau selon l'une quelconque des revendications 3 à 7, **caractérisée en ce que** ledit polymère hydrosoluble est totalement ou partiellement neutralisé, préférentiellement par les ions sodium, calcium, potassium, magnésium, ammonium ou leurs mélanges.

**9.** Suspension aqueuse de talc dans l'eau selon l'une quelconque des revendications 3 à 8, **caractérisée en ce qu'**elle contient entre 0,001 % et 2 %, préférentiellement entre 0,1 % et 1 %, très préférentiellement entre 0,1 % et 0,75 %, en poids sec dudit polymère hydrosoluble par rapport au poids sec de talc.

**10.** Suspension aqueuse de talc dans l'eau selon l'une des revendications 3 à 9, **caractérisée en ce qu'**elle contient plus de 55 %, préférentiellement plus de 60 %, très préférentiellement entre 61 % et 65 % en poids sec de talc par rapport à son poids total.

**11.** Utilisation d'une suspension aqueuse de talc dans l'eau, selon l'une des revendications 3 à 10, pour fabriquer une sauce de couchage papetière, un additif permettant de réduire la quantité de matières collantes, une peinture, un plastique, un caoutchouc, une céramique, une composition alimentaire, une composition cosmétique, une formulation agricole.

**12.** Sauce de couchage papetière, additif permettant de réduire la quantité de matières collantes, peinture, plastique, caoutchouc, céramique, composition alimentaire, composition cosmétique ou formulation agricole, **caractérisé en ce qu'**il/elle contient un talc et un polymère hydrosoluble constitué :

a) d'au moins un monomère (méth)acrylique, et
b) d'au moins un monomère de formule (I) :

$$R - (OE)m - (OP)_n - R' \qquad (I)$$

avec :

- m et n désignant des entiers inférieurs ou égaux à 115, dont un au moins est non nul,
- OE et OP désignant respectivement l'oxyde d'éthylène et l'oxyde de propylène,
- R désignant une fonction polymérisable, choisie parmi la fonction acrylate, méthacrylate, méthacryluréthane, allyl ou vinyl,
- R' désignant l'hydrogène.

## Patentansprüche

1. Verfahren zur Herstellung einer wässrigen Talkumsuspension, umfassend einen Schritt des Suspendierens eines Talkums in Wasser, wobei das Talkum gegebenenfalls aus einem vorausgegangenen Granulierungsschritt stammt, **dadurch gekennzeichnet, dass** man während des Suspendierungsschritts und/oder während des optionalen Granulierungsschritts mindestens ein wasserlösliches Polymer einsetzt, das **dadurch gekennzeichnet ist, dass** es aus Folgendem besteht:

    a) mindestens einem (Meth)Acrylmonomer und
    b) mindestens einem Monomer der Formel (I):

$$R - (OE)m - (OP)_n - R' \qquad (I)$$

wobei:

- m und n ganze Zahlen kleiner als oder gleich 115 darstellen, von denen mindestens eine ungleich null ist,
- OE und OP für Ethylenoxid bzw. Propylenoxid stehen,
- R eine polymerisierbare Funktion darstellt, die aus der Acrylat-, Methacrylat-, Methacrylurethan-, Vinyl- oder Allylfunktion ausgewählt ist,
- R' Wasserstoff bedeutet.

2. Verfahren nach Anspruch 1, **dadurch gekennzeichnet, dass** für das Monomer der Formel (I) die ganzen Zahlen m und n ungleich null und zwischen 10 und 75 sind.

3. Wässrige Talkumsuspension in Wasser, **dadurch gekennzeichnet, dass** sie mindestens ein wasserlösliches Polymer enthält, bestehend aus:

    a) mindestens einem (Meth)Acrylmonomer und
    b) mindestens einem Monomer der Formel (I):

$$R - (OE)m - (OP)_n - R' \qquad (I)$$

wobei:

- m und n ganze Zahlen kleiner als oder gleich 115 darstellen, von denen mindestens eine ungleich null ist,
- OE und OP für Ethylenoxid bzw. Propylenoxid stehen,
- R eine polymerisierbare Funktion darstellt, die aus der Acrylat-, Methacrylat-, Methacrylurethan-, Vinyl- oder Allylfunktion ausgewählt ist,
- R' Wasserstoff bedeutet.

4. Wässrige Talkumsuspension in Wasser nach Anspruch 3, **dadurch gekennzeichnet, dass** das wasserlösliche Polymer, ausgedrückt in Gew.-% seiner jeweiligen Monomere, aus Folgendem besteht:

    a) mindestens 60% mindestens eines (Meth)Acrylmonomers,

b) höchstens 40% mindestens eines Monomers der Formel (I).

5. Wässrige Talkumsuspension in Wasser nach einem der Ansprüche 3 und 4, **dadurch gekennzeichnet, dass** das wasserlösliche Polymer, ausgedrückt in Gew.-% seiner jeweiligen Monomere, aus Folgendem besteht:

a) 60% bis 99% mindestens eines (Meth)Acrylmonomers und
b) 1% bis 40% eines Monomers der Formel (I).

6. Wässrige Talkumsuspension in Wasser nach einem der Ansprüche 3 bis 5, **dadurch gekennzeichnet, dass** für das Monomer der Formel (I) des wasserlöslichen Polymers m und n ungleich null und zwischen 10 und 75 sind.

7. Wässrige Talkumsuspension in Wasser nach einem der Ansprüche 3 bis 6, **dadurch gekennzeichnet, dass** das wasserlösliche Polymer eine spezifische Viskosität zwischen 1 und 50 aufweist.

8. Wässrige Talkumsuspension in Wasser nach einem der Ansprüche 3 bis 7, **dadurch gekennzeichnet, dass** das wasserlösliche Polymer vollständig oder teilweise neutralisiert ist, vorzugsweise durch Natrium-, Calcium-, Kalium-, Magnesium-, Ammoniumionen oder deren Gemische.

9. Wässrige Talkumsuspension in Wasser nach einem der Ansprüche 3 bis 8, **dadurch gekennzeichnet, dass** sie zwischen 0,001% und 2%, vorzugsweise zwischen 0,1% und 1%, ganz besonders bevorzugt zwischen 0,1% und 0,75% Trockengewicht des wasserlöslichen Polymers, bezogen auf das Trockengewicht von Talkum, enthält.

10. Wässrige Talkumsuspension in Wasser nach einem der Ansprüche 3 bis 9, **dadurch gekennzeichnet, dass** sie mehr als 55%, vorzugsweise mehr als 60%, ganz besonders bevorzugt zwischen 61% und 65% Trockengewicht Talkum, bezogen auf ihr Gesamtgewicht, enthält.

11. Verwendung einer wässrigen Talkumsuspension in Wasser nach einem der Ansprüche 3 bis 10 zur Herstellung einer Papierstreichmasse, eines Additivs, das eine Verringerung der Menge an Klebstoffen gestattet, eines Anstrichs, eines Kunststoffs, eines Kautschuks, einer Keramik, einer Lebensmittelzusammensetzung, einer Kosmetikzusammensetzung oder einer landwirtschaftlichen Formulierung.

12. Papierstreichmasse, Additiv, das eine Verringerung der Menge an Klebstoffen gestattet, Anstrich, Kunststoff, Kautschuk, Keramik, Lebensmittelzusammensetzung, Kosmetikzusammensetzung oder landwirtschaftliche Formulierung, **dadurch gekennzeichnet, dass** sie/es/er ein Talkum und ein wasserlösliches Polymer enthält, das aus Folgendem besteht:

a) mindestens einem (Meth)Acrylmonomer und
b) mindestens einem Monomer der Formel (I):

$$R - (OE)m - (OP)_n - R' \qquad (I)$$

wobei:

- m und n ganze Zahlen kleiner als oder gleich 115 darstellen, von denen mindestens eine ungleich null ist,
- OE und OP für Ethylenoxid bzw. Propylenoxid stehen,
- R eine polymerisierbare Funktion darstellt, die aus der Acrylat-, Methacrylat-, Methacrylurethan-, Vinyl- oder Allylfunktion ausgewählt ist,
- R' Wasserstoff bedeutet.

## Claims

1. Method of manufacture of an aqueous suspension of talc, including a step of suspending in water of a talc, said talc may result from a prior step of granulation, **characterised in that** at least one hydrosoluble polymer is implemented, during the step of suspending and/or during optionally the step of granulation, **characterised in that** it is made of:

a) of at least one (meth)acrylic monomer, and
b) of at least one monomer of formula (I):

$$R - (OE)m - (OP)_n - R' \qquad (I)$$

where :

- m and n designate integers of less than or equal to 115, at least one of which is non-zero,
- OE and OP designate respectively ethylene oxide and propylene oxide,
- R designates a polymerizable group, chosen from among the acrylate, methacrylate, methacrylurethane, vinyl or allyl groups,
- R' designates hydrogen.

2. Method according to claim 1, **characterised in that**, for the monomer of formula (I), integers m and n are non-zero and between 10 and 75.

3. Aqueous suspension of talc in water, **characterised in that** it contains at least one hydrosoluble polymer consisting:

a) of at least one (meth)acrylic monomer, and
b) of at least one monomer of formula (I):

$$R - (OE)m - (OP)_n - R' \qquad (I)$$

where :

- m and n designate integers of less than or equal to 115, at least one of which is non-zero,
- OE and OP designate respectively ethylene oxide and propylene oxide,
- R designates a polymerizable group, chosen from among the acrylate, methacrylate, methacrylurethane, vinyl or allyl groups,
- R' designates hydrogen.

4. Aqueous suspension of talc in water according to claim 3, **characterised in that**, said hydrosoluble polymer is made of, expressed as a % by weight of each of its monomers:

a) at least 60% of at least one (meth)acrylic monomer,
b) at most 40% of at least one monomer of formula (I).

5. Aqueous suspension of talc in water according to any one of the claims 3 and 4, **characterised in that**, said hydrosoluble polymer is made of, expressed as a % by weight of each of its monomers:

a) of 60% to 99% of at least one (meth)acrylic monomer, and
b) of 1 % to 40% of a monomer of formula (I).

6. Aqueous suspension of talc in water according to one of the claims 3 to 5, **characterised in that**, in respect of the monomer of formula (I) of the said hydrosoluble polymer, m and n are non-zero and between 10 and 75.

7. Aqueous suspension of talc in water according to one of the claims 3 to 6, **characterised in that**, said hydrosoluble polymer has a specific viscosity of between 1 and 50.

8. Aqueous suspension of talc in water according to any one of the claims 3 to 7, **characterised in that**, said hydrosoluble polymer is totally or partially neutralised, preferentially by the sodium, calcium, potassium, magnesium and ammonium ions or their blends.

9. Aqueous suspension of talc in water according to any one of the claims 3 to 8, **characterised in that** it contains between 0.001% and 2%, preferentially between 0.1% and 1%, and very preferentially between 0.1% and 0.75%, by dry weight of the said hydrosoluble polymer relative to the dry weight of talc.

10. Aqueous suspension of talc in water according to one of the claims 3 to 9, **characterised in that** it contains more than 55%, preferentially more than 60%, and very preferentially between 61% and 65%, by dry weight of talc relative to its total weight.

**11.** Use of the said aqueous suspension of talc in water, according to one of the claims 3 to 10, to manufacture a paper coating color, an additive enabling the quantity of adhesives to be reduced, a paint, a plastic, a rubber, a ceramic, a food composition, a cosmetic composition, an agricultural formulation.

**12.** Paper coating color, an additive enabling the quantity of adhesives to be reduced, a paint, a plastic, a rubber, a ceramic, a food formulation, a cosmetic formulation, or an agricultural formulation, **characterised in that** it contains a talc and a hydrosoluble polymer consisting:

  a) of at least one (meth)acrylic monomer, and
  b) of at least one monomer of formula (I):

$$R - (OE)m - (OP)_n - R'\qquad(I)$$

where :

  - m and n designate integers of less than or equal to 115, at least one of which is non-zero,
  - OE and OP designate respectively ethylene oxide and propylene oxide,
  - R designates a polymerizable group, chosen from among the acrylate, methacrylate, methacrylurethane, allyl or vinyl groups,
  - R' designates hydrogen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1074293 A1
- JP 63175197 A
- JP 2253836 A
- FR 2380065 A1
- US 6267811 A1
- WO 2010055191 A1
- GB 2019822 A
- EP 0892020 A1
- US 6946510 B

**Littérature non-brevet citée dans la description**

- The importance of surface energy in the dispersion behaviour of talc particles in aqueous media. *Powder Technology,* 2009, vol. 190 (1-2), 242-246